# EUROPEAN PATENT APPLICATION

(11) **EP 4 027 349 A1**
(43) Date of publication of application: **13.07.2022**
(21) Application number: 20880529.1
(22) Date of filing: 03.09.2020
(51) Int. Cl.: G16H 10/00

(54) **IMAGE MANAGEMENT SYSTEM, WEARABLE DEVICE, IMAGE MANAGEMENT METHOD, AND IMAGE MANAGEMENT PROGRAM**

(30) Priority: 01.11.2019 JP 2019199662
(71) Applicant: TERUMO Kabushiki Kaisha, Tokyo 151-0072 (JP)
(72) Inventor: UTSUGIDA, Tomoki, Ashigarakami-gun, Kanagawa 259-0151 (JP); ARITA, Eiji, Ashigarakami-gun, Kanagawa 259-0151 (JP)
(74) Representative: Casalonga
(86) International application number: PCT/JP2020/033440
(87) International publication number: WO 2021/084903

(57) **Abstract**

The invention relates to an image management system, a wearable device, an image management method, and an image management program. An image management system (10) includes a wearable device (12) including a camera (48) and an information processing server (16). The information processing server (16) includes a server storage unit (100) that stores captured image received from the wearable device (12). One of the wearable device (12) and the information processing server (16) includes an information organization unit. The information organization unit deletes the captured image when an image determination unit determines that the captured image is not a meal image, and the server storage unit (100) holds the captured image when the image determination unit determines that the captured image is the meal image.

## Description

### Technical Field

The present invention relates to an image management system, a wearable device, an image management method, and an image management program.

### Background Art

JP-A-2019-28625 discloses an image management system that transmits a captured image (meal image) captured by a camera of a user terminal such as a smartphone to a server and stores the captured image (meal image) in the server. The captured image stored in the server is used for blood glucose management (diabetes treatment guidance, health guidance, or the like).

### Summary of Invention

However, in the image management system as described above, when the user terminal is forgotten to be carried or the user terminal is in a bag, image-capturing of the meal cannot be quickly performed. In addition, even when the captured image is not a meal image, because the captured image is stored in the server as it is, there is a concern that health management (blood glucose management) using the meal image cannot be performed effectively.

The invention has been made in consideration of such a problem, and an object is to provide an image management system, wearable device, image management method, and image management program capable of efficiently capturing an image of a meal and effectively performing health management using a meal image.

A first aspect of the invention is an image management system for medical use including: a wearable device worn by a use; and an information processing server communicatable with the wearable device, wherein the wearable device includes a camera for image-capturing a subject, wherein the information processing server includes a server storage unit that stores a captured image received from the wearable device, wherein any one of the wearable device and the information processing server includes an information organization unit that organizes the captured image based on a determination result of an image determination unit as to whether or not the captured image taken by the camera is a meal image, wherein the information organization unit deletes the captured image when the image determination unit determines that the captured image is not the meal image, and wherein the server storage unit holds the captured image when the image determination unit determines that the captured image is the meal image.

A second aspect of the invention is a wearable device for medical use worn by a user, including: a camera for image-capturing a subject; a storage unit that stores a captured image taken by the camera; a determination communication unit that transmits the captured image stored in the storage unit to an image determination server and receives a determination result of the image determination server as to whether or not the captured image is a meal image; and a device information organization unit that organizes the captured image stored in the storage unit, wherein the device information organization unit deletes the captured image from the storage unit when the determination communication unit receives the determination result indicating that the captured image is not the meal image and deletes the captured image from the storage unit after transmitting the captured image to an information processing server when the determination communication unit receives the determination result indicating that the captured image is the meal image.

A third aspect of the invention is an image management method for medical use including: an image-capturing step of image-capturing a subject by a camera of a wearable device worn by a user; an image transmitting step of transmitting a captured image taken in the image-capturing step to an information processing server; a storing step of storing the captured image received from the wearable device in a server storage unit of the information processing server; and a server information organizing step of organizing the captured image based on the determination result of an image determination unit as to whether or not the captured image taken by the camera is a meal image, wherein, in the server information organizing step, when the image determination unit determines that the captured image is not the meal image, the captured image is deleted, and when the image determination unit determines that the captured image is the meal image, the captured image is held in the server storage unit.

A fourth aspect of the invention is an image management method for medical use including: an image-capturing step of image-capturing a subject by a camera of a wearable device worn by a user; a storing step of storing a captured image taken in the image-capturing step in a storage unit of the wearable device; an image determination step of transmitting the captured image stored in the storage unit to an image determination server and receiving a determination result of the image determination server as to whether or not the captured image is a meal image; and an information organizing step of organizing the captured image stored in the storage unit, wherein, in the information organizing step, the captured image is deleted from the storage unit when the wearable device receives the determination result indicating that the captured image is not the meal image, and the captured image is deleted after the captured image is transmitted to an information processing server when the wearable device receives the determination result indicating that the captured image is the meal image.

A fifth aspect of the invention is an image management program for medical use, causing a computer to execute: an image capturing step of image-capturing a subject by a camera of a wearable device worn by a user; an image transmitting step of transmitting a captured image taken in the image-capturing step to an information processing server; a storing step of storing the captured image received from the wearable device in a server storage unit of the information processing server; and a server information organizing step of organizing the captured image based on a determination result of an image determination unit as to whether or not the captured image taken by the camera is a meal image, wherein, in the server information organizing step, the captured image is deleted when the image determination unit determines that the captured image is not the meal image, and the captured image is held in the server storage unit when the image determination unit determines that the captured image is the meal image.

A sixth aspect of the invention is an image management program for medical use, causing a computer to execute: an image-capturing step of image-capturing a subject with a camera of a wearable device worn by a user; a storing step of storing a captured image taken in the image-capturing step in a storage unit of the wearable device; an image determination step of transmitting the captured image stored in the storage unit to an image determination server and receiving a determination result of the image determination server as to whether or not the captured image is a meal image; and an information organizing step of organizing the captured image stored in the storage unit, wherein, in the information organizing step, the captured image is deleted from the storage unit when the wearable device receives the determination result indicating that the captured image is not the meal image, and the captured image is deleted after the captured image is transmitted to an image processing server when the wearable device receives the determination result indicating that the captured image is the meal image.

According to the invention, when the user wears the wearable device, the image-capturing of the meal by the camera of the wearable device can be quickly performed. Accordingly, it is possible to efficiently capture an image of the meal. In addition, because the captured image is deleted from the server storage unit (storage unit) when the captured image is not the meal image, health management using the meal image can be effectively performed.

### Brief Description of Drawings

[FIG. 1] FIG. 1 is a block diagram of an image management system according to an embodiment of the invention.
[FIG. 2] FIG. 2 is a perspective view of a wearable device of FIG. 1.
[FIG. 3] FIG. 3A is a front view of the wearable device of FIG. 2, and FIG. 3B is a vertical sectional view taken along the line IIIB-IIIB of FIG. 3A.
[FIG. 4] FIG. 4 is a block diagram of a device main body forming the wearable device of FIG. 3B.
[FIG. 5] FIG. 5 is a flowchart illustrating an operation of the wearable device of FIG. 1.
[FIG. 6] FIG. 6 is a first flowchart illustrating an operation of an information processing server of FIG. 1.
[FIG. 7] FIG. 7 is a second flowchart illustrating an operation of the information processing server of FIG. 1.
[FIG. 8] FIG. 8 is an example of a blood glucose management graph.
[FIG. 9] FIG. 9 is a flowchart according to Modified Example 1 illustrating an operation of the wearable device of FIG. 1.
[FIG. 10] FIG. 10 is a flowchart according to Modified Example 2 illustrating an operation of the wearable device of FIG. 1.
[FIG. 11] FIG. 11A is a first explanatory view of image-capturing a subject by a camera of the wearable device, and FIG. 11B is a second explanatory view of image-capturing the subject by the camera of the wearable device.
[FIG. 12] FIG. 12 is a block diagram illustrating a wearable device according to Modified Example.
[FIG. 13] FIG. 13 is a flowchart illustrating an operation of the wearable device of FIG. 12.

### Description of Embodiments

Hereinafter, preferred embodiments of an image management system, a wearable device, an image management method, and an image management program according to the invention will be described with reference to the accompanying drawings.

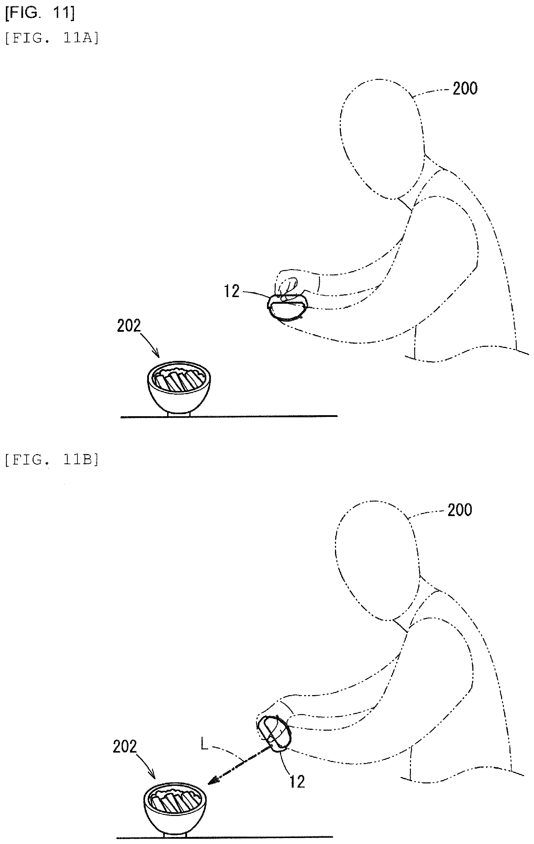

An image management system 10 according to an embodiment of the invention is a medical system for performing health management (for example, blood glucose management) using a meal image captured by a user 200 (refer to FIG. 11A).

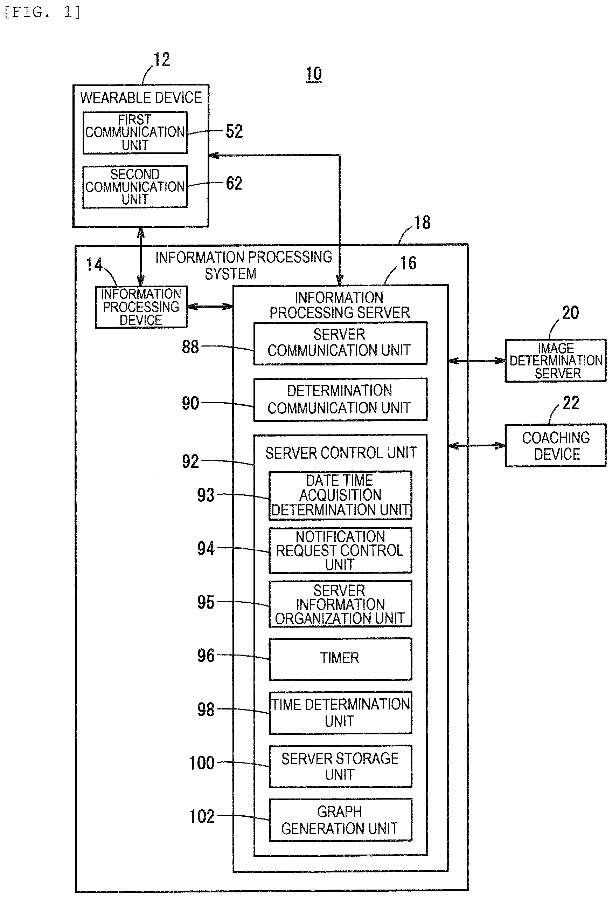

As illustrated in FIG. 1, the image management system 10 includes a wearable device 12, an information processing system 18 having an information processing device 14 and an information processing server 16, an image determination server 20, and a coaching device 22.

As illustrated in FIG. 11A, the wearable device 12 is configured to be worn by the user 200. The wearable device 12 is, for example, configured to be detachable from a wrist like a wristband. However, the wearable device 12 may be integrally provided on the spectacles, may be configured to be detachable from clothes, may be configured to be detachable from the neck like a necklace, or may be detachable from the head. That is, the wearable device 12 may be configured in any manner as long as the user 200 can wear the wearable device detachably.

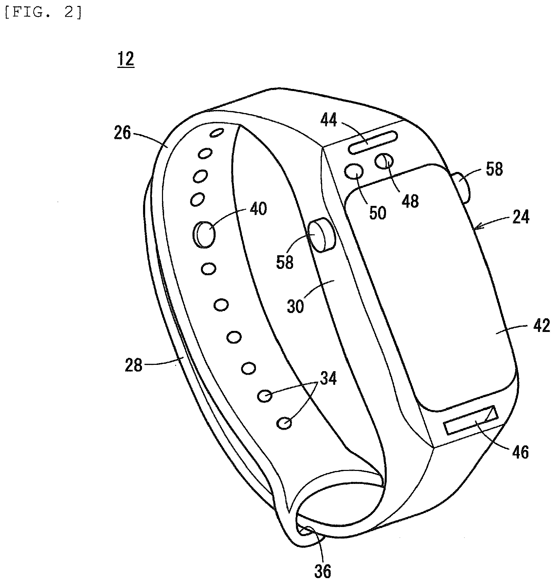

In FIGS. 2 to 3B, the wearable device 12 includes a device main body 24 and a first belt portion 26 and a second belt portion 28 for attaching the device main body 24 to the wrist of the user 200. The device main body 24 has a case 30 formed in a vertically long box shape. However, the case 30 may have a horizontally long box shape, a box shape (other than a square shape) having a polygonal bottom surface, a columnar shape, an elliptical columnar shape, or the like.

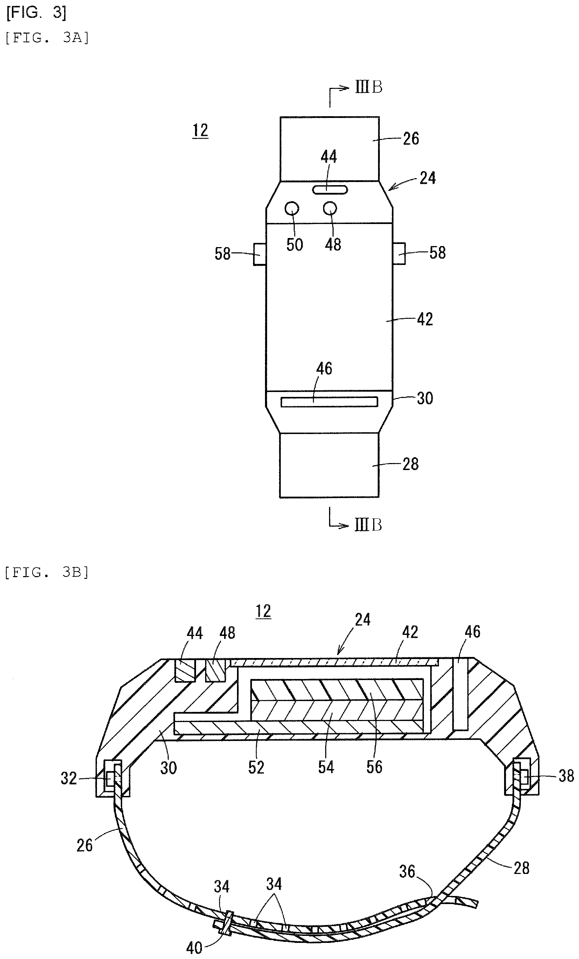

As illustrated in FIGS. 2 and 3B, the first belt portion 26 is fixed to one end of the case 30 by a first fixing portion 32. A plurality of locking holes 34 are formed in the first belt portion 26. The plurality of locking holes 34 are arranged at equal intervals in an extending direction of the first belt portion 26. A belt through hole 36 for passing the second belt portion 28 is provided at a tip end portion of the first belt portion 26.

The second belt portion 28 is fixed to the other end of the case 30 by a second fixing portion 38. A locking pin 40 that can be fitted into the locking hole 34 of the first belt portion 26 is provided at the tip of the second belt portion 28. The first belt portion 26 and the second belt portion 28 are locked to each other by fitting the locking pin 40 into the locking hole 34.

In FIGS. 2 to 3B, the device main body 24 has a display unit 42, an indicator 44, a blood glucose sensor mounting unit 46, a camera 48, a light emitting unit 50, a first communication unit 52, a battery 54, a printed circuit board (PCB) 56, and two operation switches 58.

The display unit 42 is provided on the surface side of the case 30. The display unit 42 can be configured with an LED display, an LCD display, a CRT display, a plasma display, a touch screen display, or the like, but the invention is not limited thereto.

The indicator 44 is provided on the side of the case 30 where the first fixing portion 32 is located with respect to the display unit 42. The indicator 44 is for displaying the state of the device main body 24 and is configured as an LED indicator.

The blood glucose sensor mounting unit 46 is provided on the side of the case 30 where the second fixing portion 38 is located with respect to the display unit 42. A blood glucose sensor (not illustrated) into which the blood is taken is attached to the blood glucose sensor mounting unit 46.

The camera 48 is for image-capturing the meal of the user 200. The camera 48 is provided on the side of the case 30 where the first fixing portion 32 is located with respect to the display unit 42. The camera 48 is configured as a pinhole camera. However, the camera 48 is not limited to the pinhole camera, and the camera may be a camera with a lens.

In FIGS. 2 and 3A, the light emitting unit 50 is provided at a position different from that of the camera 48 in the case 30 so as not to interfere with image-capturing function of the camera 48. Specifically, the light emitting unit 50 is provided on the side of the camera 48. The light emitting unit 50 outputs a guide light L indicating an image-capturing orientation of the camera 48 (refer to FIG. 11B). The guide light L is visible light, but the color thereof can be appropriately set. An emission intensity of the guide light L may be such that an area within 1 m from the light emitting unit 50 can be irradiated.

The light emitting unit 50 has, for example, a laser diode that oscillates a laser beam as the guide light L. In this case, the guide light L output from the laser diode passes through the aperture formed on the mask and is output from a projection optical system toward a subject 202 (refer to FIGS. 11A and 11B). The shape of the aperture can be set as appropriate. The light (guide light L) oscillated from the laser diode irradiates one point of the subject 202.

The light emitting unit 50 may have a light emitting element such as an LED element. In this case, the guide light L output from the light emitting unit 50 spreads toward the subject 202. The light emitting element is not limited to the LED element, and may be an organic EL element, an inorganic EL element, or the like. The guide light L emitted from the light emitting unit 50 also functions as an AF auxiliary light of the camera 48.

In FIG. 3B, the first communication unit 52 is housed in the case 30. The first communication unit 52 constructs a wireless communication line between the first communication unit 52 and a server communication unit 88 (refer to FIG. 1) of the information processing server 16 to perform information communication. LPWA is preferable as the standard of the wireless communication line between the first communication unit 52 and the server communication unit 88. However, the standard of the wireless communication line between the first communication unit 52 and the server communication unit 88 may be, for example, Wi-Fi (registered trademark), LTE (registered trademark), or the like.

The battery 54 is housed in the case 30 so as to be located closer to the display unit 42 than the first communication unit 52. The battery 54 supplies power to the electronic components of the device main body 24. The battery 54 includes a secondary battery, a capacitor, and the like. The printed circuit board 56 is housed in the case 30 so as to be located closer to the display unit 42 than the battery 54. Electronic components (not illustrated) are mounted on the printed circuit board 56.

In FIGS. 2 and 3A, the two operation switches 58 (operation units) are for image-capturing by the camera 48 and are provided on the right and left side surfaces of the case 30 one by one. Each operation switch 58 is a push button type switch. However, each operation switch 58 is not limited to the push button type, and a slide switch or the like may be used.

In the device main body 24, the arrangement of the indicator 44, the blood glucose sensor mounting unit 46, the first communication unit 52, the battery 54, and the printed circuit board 56 can be appropriately changed. The number, size, shape, and position of the operation switches 58 can be appropriately set.

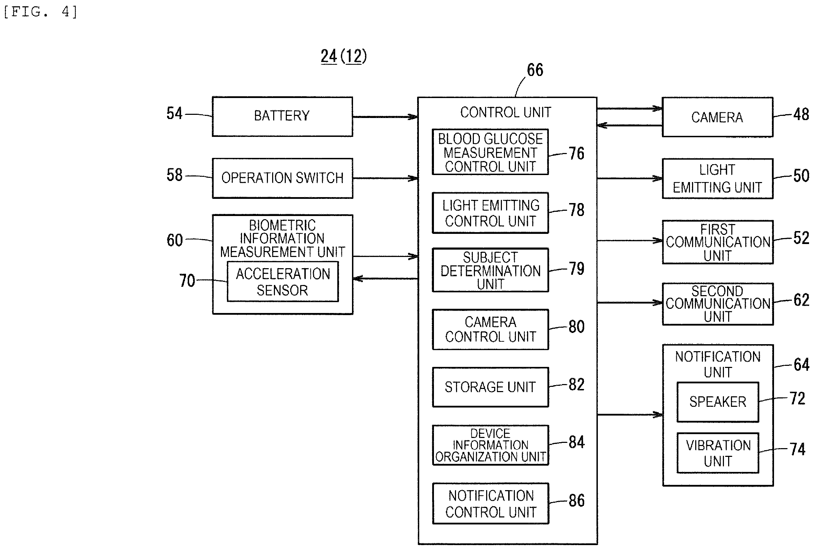

As illustrated in FIG. 4, the device main body 24 of the wearable device 12 further includes a biometric information measurement unit 60, a second communication unit 62, a notification unit 64, and a control unit 66.

The biometric information measurement unit 60 measures biometric information of the user 200. Specifically, the biometric information measurement unit 60 has an acceleration sensor 70. The acceleration sensor 70 measures, for example, the number of steps of the user 200. The biometric information measurement unit 60 may further measure blood pressure, pulse (heart rate), body temperature, and the like. In this case, the biometric information measurement unit 60 may include for example, a heart rate detection sensor. The heart rate detection sensor measures the heart rate by any of an electrocardiogram method, a photoelectric pulse wave method, a blood pressure measurement method, and a phonocardiogram method.

The information measured by the biometric information measurement unit 60 is displayed on the display unit 42. Specifically, the display unit 42 displays a blood glucose level (glucose concentration), the amount of activity, the number of steps, the heart rate, the sleep time, and the like. In addition, the display unit 42 displays the current date and time, whether or not the biometric information can be measured, whether or not the data (for example, the captured image by the camera 48) can be transmitted, and the like. It is noted that the captured image itself taken by the camera 48 is not displayed on the display unit 42.

As illustrated in FIG. 1, the second communication unit 62 constructs a wireless communication line between the second communication unit 62 and the information processing device 14 to perform information communication. The standard of the wireless communication line between the second communication unit 62 and the information processing device 14 is Bluetooth (registered trademark), and BLE (Bluetooth Low Energy) is particularly preferable. However, the standard of the wireless communication line between the second communication unit 62 and the information processing device 14 may be, for example, Wi-Fi (registered trademark) or the like. In addition, the second communication unit 62 may construct a wired communication line between the second communication unit 62 and the information processing device 14 to perform information communication. The second communication unit 62 transmits, for example, the biometric information (step count information, or the like) to the information processing device 14.

In FIG. 4, the notification unit 64 includes a speaker 72 and a vibration unit 74. The speaker 72 outputs sound information (a voice, an electronic sound, or the like). The vibration unit 74 transmits the vibration to the user 200.

The control unit 66 is a computer including a microcomputer, and the control unit has a central processing unit (CPU), a ROM and a RAM, as a memory, and the like and functions as a function realization unit (function realization means) by the CPU reading and executing a program stored in the ROM. It is noted that the various function realization units can also be configured by a function realizer as hardware.

The control unit 66 includes a blood glucose measurement control unit 76, a light emitting control unit 78, a subject determination unit 79, a camera control unit 80, a storage unit 82, a device information organization unit 84, and a notification control unit 86.

The blood glucose measurement control unit 76 measures the blood glucose level (glucose concentration in plasma) of the blood taken into the blood glucose measurement sensor. A continuous glucose monitor may be used when blood glucose information is to be continuously acquired. In this case, the blood glucose measurement control unit 76 acquires a measured value and measured time information from the continuous glucose monitor. The light emitting control unit 78 controls an operation of the light emitting unit 50 to output the guide light L from the light emitting unit 50. The subject determination unit 79 determines whether or not the subject 202 within the image-capturing range of the camera 48 is the same as or similar to a predetermined meal image. The camera control unit 80 controls an operation of the camera 48.

The storage unit 82 stores the blood glucose information (measured value and measurement date and time acquired by the blood glucose measurement control unit 76), the captured image by the camera 48, and the like. The device information organization unit 84 organizes the captured image stored in the storage unit 82. The notification control unit 86 controls an operation of the notification unit 64. Specifically, the notification control unit 86 outputs sound information from the speaker 72 or vibrates the vibration unit 74.

As illustrated in FIG. 1, the information processing device 14 is operated by the user 200. The information processing device 14 is, for example, a smartphone, a laptop, a tablet, or the like, but the invention is not limited thereto. The information processing device 14 transmits, for example, setting instruction information and operation instruction information of the wearable device 12 to the second communication unit 62 of the wearable device 12.

The information processing device 14 constructs a wireless communication line between the information processing device 14 and the server communication unit 88 of the information processing server 16 to perform information communication. The standard of the wireless communication line between the information processing device 14 and the server communication unit 88 may be the same as the standard of the wireless communication line between the first communication unit 52 of the wearable device 12 and the server communication unit 88 of the information processing server 16. The information processing device 14 transmits, for example, the biometric information or the like to the server communication unit 88.

The information processing server 16 includes a server communication unit 88, a determination communication unit 90, and a server control unit 92. The server communication unit 88 constructs a wireless communication line between the server communication unit 88 and the coaching device 22 to perform information communication. The standard of the wireless communication line between the server communication unit 88 and the coaching device 22 may be the same as the standard of the wireless communication line between the first communication unit 52 of the wearable device 12 and the information processing server 16.

The determination communication unit 90 constructs a wireless communication line between the determination communication unit 90 and the image determination server 20 to perform information communication. The standard of the wireless communication line between the determination communication unit 90 and the image determination server 20 may be the same as the standard of the wireless communication line between the first communication unit 52 of the wearable device 12 and the server communication unit 88. It is noted that an image determination function may be added to the wearable device 12 instead of the image determination server 20. In this case, the determination communication unit 90 can be omitted.

The server control unit 92 includes a date time acquisition determination unit 93, a notification request control unit 94, a server information organization unit (information organization unit) 95, a timer 96, a time determination unit 98, a server storage unit 100, and a graph generation unit 102.

The date time acquisition determination unit 93 determines whether or not the meal date time of the user 200 has been acquired. The notification request control unit 94 requests the wearable device 12 to perform notification control for prompting to input the meal date time. The server information organization unit 95 organizes the captured image stored in the server storage unit 100.

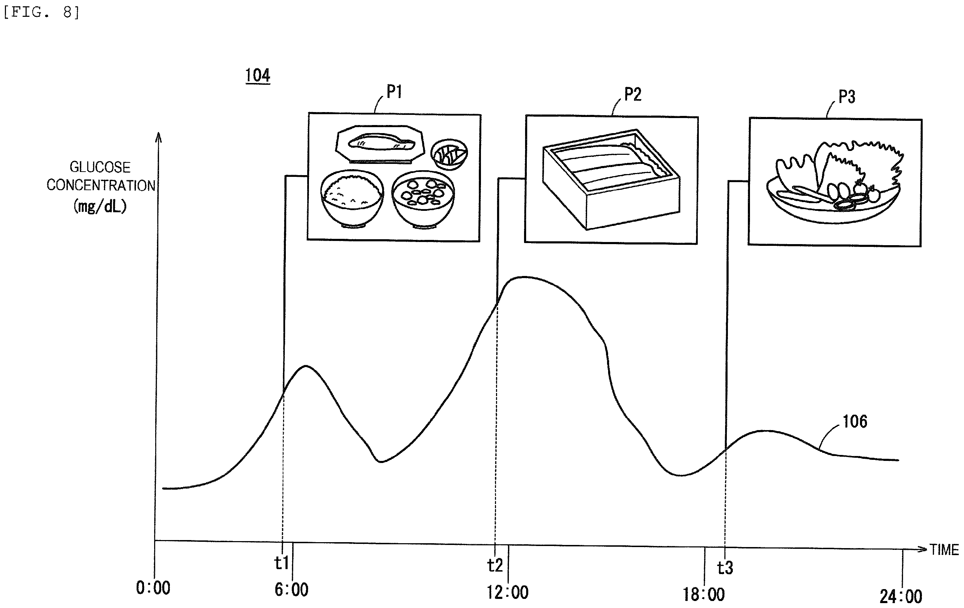

The timer 96 measures the time. The time determination unit 98 performs a predetermined time determination. The blood glucose information (blood glucose date and time and glucose concentration) and the captured image (meal image) are stored in the server storage unit 100. The graph generation unit 102 generates a blood glucose management graph 104 (refer to FIG. 8) based on the blood glucose information and meal image stored in the server storage unit 100.

The coaching device 22 is a medical device operated by a medical worker (doctor, registered dietitian, or the like) and is used to perform blood glucose management (treatment guidance, health guidance, or the like) of the user 200.

Next, an image management method using the image management system 10 will be described. First, the operation flow of the wearable device 12 will be described.

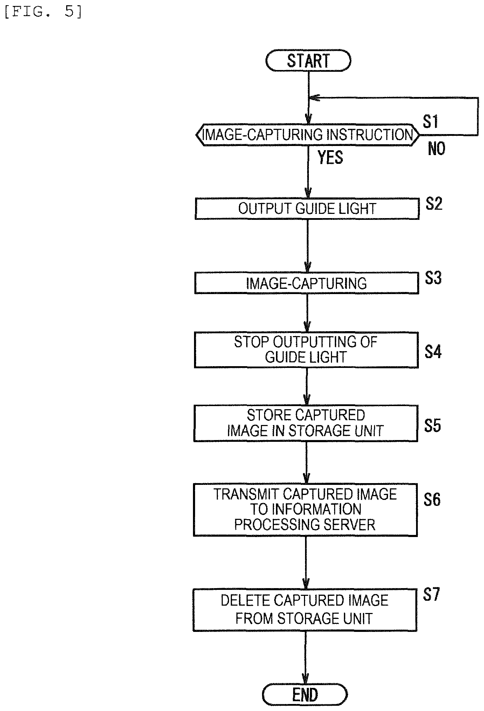

As illustrated in FIG. 5, first, the control unit 66 determines whether or not there is an image-capturing instruction (step S1). Specifically, when the two operation switches 58 are operated at the same time, the control unit 66 determines that there is an image-capturing instruction. It is noted that, when the two operation switches 58 are not operated at the same time (for example, when only one of the operation switches 58 is operated), the control unit 66 determines that there is no image-capturing instruction. When the control unit 66 determines that there is no image-capturing instruction (step S1: NO), the process remains in step S1 until image-capturing instruction is given.

When the control unit 66 determines that the image-capturing instruction has been given (step S1: YES), the light emitting control unit 78 outputs the guide light L from the light emitting unit 50 (step S2). At this time, the user 200 directs the guide light L toward the subject 202.

Subsequently, the camera control unit 80 allows the camera 48 to perform image-capturing (step S3). Specifically, the subject determination unit 79 determines whether or not the subject 202 entering the image-capturing range of the camera 48 corresponds to the predetermined meal information. The subject determination unit 79 determines, for example, whether or not the subject 202 entering the image-capturing range of the camera 48 is the same as or similar to a predetermined meal image.

Then, the camera control unit 80 prohibits the camera 48 from image-capturing the subject 202 when the subject determination unit 79 determines that the subject 202 entering the image-capturing range of the camera 48 does not correspond to the predetermined meal information. That is, because it is highly possible that the subject 202 is not the meal, the camera control unit 80 does not allow the camera 48 to perform image-capturing the subject 202 (does not release the shutter).

On the other hand, the camera control unit 80 permits the camera 48 to image-capture the subject 202 when the subject determination unit 79 determines that the subject 202 entering the image-capturing range of the camera 48 corresponds to the predetermined meal information. That is, because the subject 202 is likely to be the meal, the camera control unit 80 allows the camera 48 to perform image-capturing the subject 202 (releases the shutter). That is, the camera control unit 80 captures an image of the subject 202 when the subject 202 is the meal.

In step S3, when the image-capturing by the camera 48 is completed, the camera control unit 80 may output an image-capturing sound (for example, a shutter sound) from the speaker 72 or blink the indicator 44. By doing so, the user 200 can easily know that the image-capturing is completed.

Next, the light emitting control unit 78 stops the outputting of the guide light L from the light emitting unit 50 (step S4). That is, when the image-capturing of the subject 202 is completed, the outputting of the guide light L is automatically stopped without operating the operation switches 58. Accordingly, the user 200 can more easily know that the image-capturing is completed. After that, the device information organization unit 84 stores the captured image in the storage unit 82 (step S5).

Then, the wearable device 12 transmits the captured image stored in the storage unit 82 to the information processing server 16 (step S6). Specifically, the first communication unit 52 transmits the captured image stored in the storage unit 82 to the information processing server 16. In step S6, the information processing device 14 may transmit the captured image to the information processing server 16 after the second communication unit 62 transmits the captured image stored in the storage unit 82 to the information processing device 14. By doing so, in comparison with the case where the first communication unit 52 directly transmits the captured image to the information processing server 16, the consumption of the battery 54 of the wearable device 12 can be prevented.

Subsequently, the device information organization unit 84 deletes the captured image from the storage unit 82 (step S7). Accordingly, the storage capacity of the storage unit 82 required to store the captured image can be reduced. After that, the operation flow of the wearable device 12 is terminated.

Next, the operation flow of the information processing server 16 will be described.

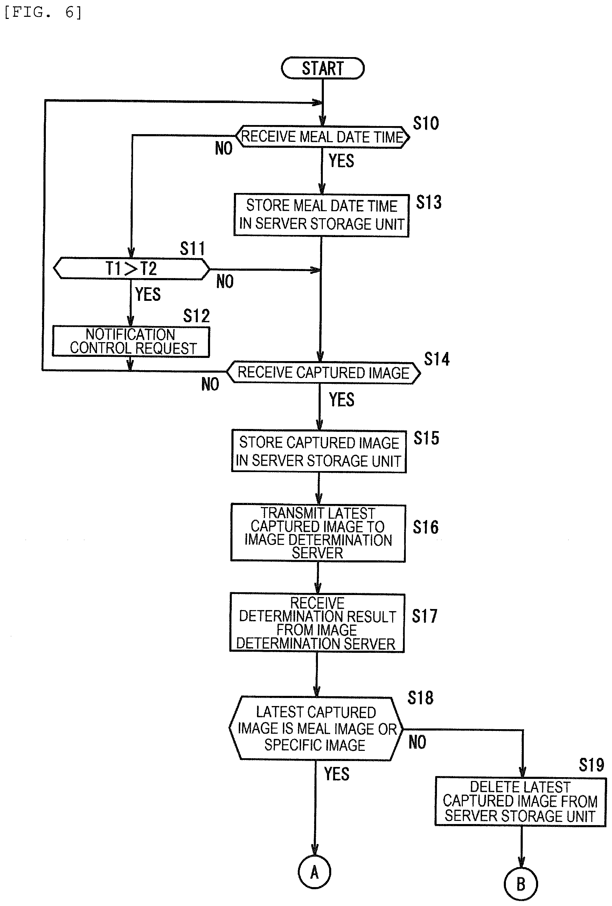

As illustrated in FIG. 6, the date time acquisition determination unit 93 determines whether or not the server communication unit 88 has received meal date time (step S10). The meal date time is a date and time (meal start date and time) when the user 200 actually ate the meal, and is, for example, registered in the information processing device 14 by the user 200. The meal date time registered in the information processing device 14 is transmitted from the information processing device 14 to the information processing server 16.

When the date time acquisition determination unit 93 determines that the meal date time has not been received (step S10: NO), the time determination unit 98 determines whether or not an elapsed time T1 from the previous meal date time exceeds a predetermined meal interval T2 (step S11). Herein, the meal interval T2 is set to, for example, 6 hours when the previous meal date time is breakfast or lunch time and 12 hours when the previous meal date time is dinner time. However, the meal interval T2 can be set as appropriate.

When the time determination unit 98 determines that the elapsed time T1 from the previous meal date time has not reached the predetermined meal interval T2 (step S11: NO), the process proceeds to step S14 described later.

When the time determination unit 98 determines that the elapsed time T1 from the previous meal date time exceeds the predetermined meal interval T2 (step S11: YES), the server communication unit 88 transmits notification control request to the wearable device 12 (step S12). When the first communication unit 52 of the wearable device 12 receives the notification control request, the notification control unit 86 performs notification control for prompting the user 200 to register the meal date time. Specifically, the notification control unit 86 outputs, for example, a voice "please register the meal date time" from the speaker 72. In addition, instead of (or in addition to) the notification by the voice, the display unit 42 may be configured to display a figure or an image for prompting to register the meal date time. It is noted that, at this time, the notification control unit 86 may vibrate the vibration unit 74.

In addition, in step S12, the server communication unit 88 may transmit the notification control request to the information processing device 14. In this case, when the information processing device 14 receives the notification control request, the information processing device 14 performs notification control for prompting the user 200 to register the meal date time. Specifically, the information processing device 14 outputs, for example, a voice "please register the meal date time" from the speaker (not illustrated) of the information processing device 14. In addition, instead of (or in addition to) the notification by the voice, the characters "please register the meal date time" may be displayed on the display unit (not illustrated) of the information processing device 14.

Furthermore, in step S12, the server communication unit 88 may transmit the notification control request to both the wearable device 12 and the information processing device 14. In this case, notification control is performed on both the wearable device 12 and the information processing device 14. After step S12, the process returns to step S10.

When the server control unit 92 determines that the meal date time has been received (step S10: YES), the server information organization unit 95 stores the received meal date time in the server storage unit 100 (step S13). Subsequently, the server control unit 92 determines whether or not the server communication unit 88 has received the captured image (step S14). The captured image is transmitted from the first communication unit 52 to the server communication unit 88 by the wearable device 12 performing the process of step S6 in FIG. 5 described above.

When the server control unit 92 determines that the captured image has not been received (step S14: NO), the process returns to step S10. On the other hand, when the server control unit 92 determines that the captured image has been received (step S14: YES), the captured image is stored in the server storage unit 100 (step S15).

Subsequently, the determination communication unit 90 transmits the latest captured image stored in the server storage unit 100 to the image determination server 20 (step S16). Then, the image determination server 20 determines whether or not the captured image is an image including meal information and determines that the captured image is the meal image when the image includes the meal information. Then, the determination result is transmitted to the determination communication unit 90. The determination communication unit 90 receives the determination result as to whether or not the captured image is the meal image (step S17). It is noted that, in step S16, the image determination server 20 can determine whether or not the captured image includes the specific image registered in advance in the image determination server 20.

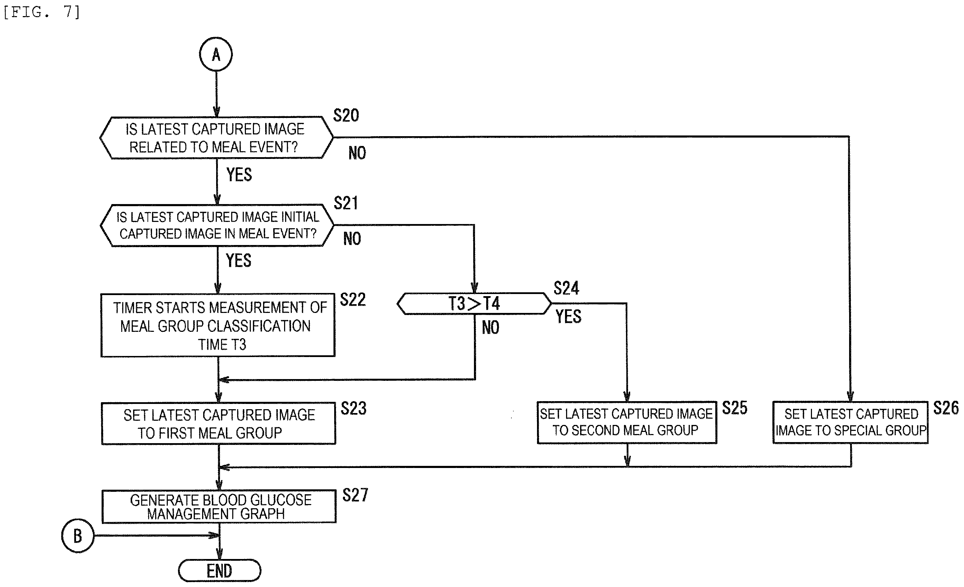

Next, when the determination communication unit 90 receives the determination result indicating that the latest captured image is neither a meal image nor a specific image (step S18: NO), the captured image is deleted from the server storage unit 100 (step S19). Accordingly, the storage capacity of the server storage unit 100 required for storing the captured image can be reduced. In addition, even when capturing an image (for example, a voyeur image or the like) that does not include meal information or a specific image is performed by the wearable device 12, the captured image can be deleted from the server storage unit 100. After that, the current operation flow is terminated (refer to FIG. 7).

When the determination communication unit 90 receives the determination result indicating that the latest captured image includes the meal image or the specific image (step S18: YES), as illustrated in FIG. 7, the server control unit 92 determines whether or not the latest captured image is related to a meal event (step S20). Specifically, when the captured image includes the meal information based on the determination of the server control unit 92, the captured image is registered as the meal image and classified as the meal event. When the server control unit 92 determines that the captured image does not include the meal information, the captured image is classified as a special event. Alternatively, the user 200 may register, in the information processing device 14, whether the event related to the image-capturing is the meal event or the special event when image-capturing by the camera 48 of the wearable device 12 is performed.

The special event includes information related to blood glucose management of the user 200 other than the meal content itself. More specifically, as the special events, exemplified are taking internal medicine, injecting insulin, fever, hypoglycemia and hyperglycemia, fatigue, presence or absence of exercise, a starting time of a meal, and an ending time of the meal. A specific figure (for example, an internal medicine being taken) indicating the special event is registered in advance in each of the information processing device 14 and the information processing server 16. When any of the registered specific figures is included in the captured image at a predetermined ratio, this situation can be determined as the special event. Accordingly, the processes of the information processing device 14 and the information processing server 16 can be simplified, and the user 200 can easily collect and record information by merely capturing an image of a preset object with the wearable device 12. It is noted that the user 200 may transmit the information manually input as to whether an event is the meal event or the special event to the information processing device 14 and the information processing server 16.

When the server control unit 92 determines that the latest captured image is related to the meal event (step S20: YES), the server control unit 92 determines whether or not the latest captured image is an initial captured image in the meal event (step S21). When the server control unit 92 determines that the latest captured image is the initial captured image in the meal event (step S21: YES), the timer 96 starts measuring a meal group classification time T3 (step S22). After that, the latest captured image is set in the first meal group (step S23).

On the other hand, when the server control unit 92 determines that the latest captured image is not the initial captured image in the meal event (that is, the latest captured image is the second and subsequent captured images in the meal event) (step S21: NO), the time determination unit 98 determines whether or not the meal group classification time T3 exceeds a predetermined time T4 (step S24). Herein, the predetermined time T4 is set to, for example, 30 minutes. However, the predetermined time T4 can be appropriately set and may be one hour.

When the time determination unit 98 determines that the meal group classification time T3 does not exceed the predetermined time T4 (step S24: NO), the latest captured image is set in the first meal group (step S23). Accordingly, it is possible to associate a plurality of the captured images taken in a relatively short time (predetermined time T4) with one meal event. After that, the process proceeds to step S27 described later.

When the time determination unit 98 determines that the meal group classification time T3 exceeds the predetermined time T4 (step S24: YES), the latest captured image is set in the second meal group (step S25). Accordingly, the captured image (meal image) acquired after a relatively long time (predetermined time T4) has elapsed because the initial captured image (meal image) of the meal event was acquired is not related to the meal event. After that, the process proceeds to step S27 described later.

When the server control unit 92 determines that the latest captured image is not related to the meal event (related to the special event) (step S20: NO), the server information organization unit 95 sets the latest captured image in the special group (step S26). The special group can be arbitrarily set by the user 200, and the corresponding specific figure may be set for each special group.

After that, the graph generation unit 102 generates the blood glucose management graph 104 based on the captured image (meal image) stored in the server storage unit 100 and the blood glucose information (step S27). Herein, the blood glucose information includes the measured value and measurement time of the glucose concentration of a sample taken into the glucose sensor. The blood glucose information is measured by the wearable device 12 and, after that, transmitted from the first communication unit 52 to the server communication unit 88. However, the blood glucose information may be transmitted from the second communication unit 62 to the server communication unit 88 through the information processing device 14.

The graph generation unit 102 generates, for example, the blood glucose management graph 104 illustrated in FIG. 8. As illustrated in FIG. 8, the blood glucose management graph 104 displays meal images P1 to P3 superimposed on the glucose concentration line 106 illustrating the time change in the measured value. It is noted that, in the blood glucose management graph 104, the horizontal axis represents the time, and the vertical axis represents the measured value (glucose concentration). In the blood glucose management graph 104, the meal image P1 is displayed at a time point t1, a meal image P2 is displayed at a time point t2, and the meal image P3 is displayed at a time point t3. In addition, at the time point when the image of the special group is acquired, an icon or the like related to the attribute of the special group can be displayed. Accordingly, the special events can be written together in a chronological order on the blood glucose management graph 104.

Such an image management method includes the image-capturing step (step S3) of image-capturing the subject 202 by the camera 48 of the wearable device 12 worn by the user 200, the image transmitting step (step S6) of transmitting the captured image taken in the image-capturing step to the information processing server 16, the storing step (step S15) of storing the captured image received from the wearable device 12 in the server storage unit 100 of the information processing server 16, and the information organizing steps (steps S19 to S26) of organizing the captured image based on the determination result of the image determination unit (image determination server 20) as to whether or not the captured image taken by the camera 48 is the meal image, and in the server information organizing step, the captured image is deleted when the image determination unit determines that the captured image is not the meal image, and the captured image is held in the server storage unit 100 when the image determination unit determines that the captured image is the meal image.

The image management method is not limited to the method described above. For example, when an image determination function is added to the wearable device 12 instead of the image determination server 20, after storing the captured image by the camera 48 in the storage unit 82 (after step S5), the control unit 66 determines whether or not the captured image stored in the storage unit 82 is the meal image or the specific image. When the captured image stored in the storage unit 82 is neither the meal image nor the specific image, the device information organization unit 84 (information organization unit) deletes the latest captured image from the storage unit 82. When the captured image stored in the storage unit 82 is the meal image or the specific image, the control unit 66 performs the same processes as in steps S20 to S26 described above and transmits the captured image to the information processing server 16.

In this case, the present embodiment obtains the following effects.

The image management system 10 includes the wearable device 12 and the information processing server 16. The wearable device 12 includes the camera 48 for image-capturing the subject 202. The information processing server 16 includes the server storage unit 100 that stores the captured image received from the wearable device 12 and the information organization unit (the device information organization unit 84 or the server information organization unit 95) that organizes the captured image based on the determination result of the image determination unit as to whether or not the captured image taken by the camera 48 is the meal image. The information organization unit deletes the captured image when the image determination unit determines that the captured image is not the meal image, and the server storage unit 100 holds the captured image in the server storage unit 100 when the image determination unit determines that the captured image is the meal image.

According to such a configuration, when the user 200 wears the wearable device 12, the image-capturing of the meal by the camera 48 of the wearable device 12 can be quickly performed. Accordingly, it is possible to efficiently capture an image of the meal. In addition, because the captured image is deleted when the captured image is not a meal image, health management using the meal image can be effectively performed.

The image determination unit is an image determination server 20 provided separately from the wearable device 12 and the information processing server 16. The information processing server 16 includes the determination communication unit 90 that transmits the captured image stored in the server storage unit 100 to the image determination server 20 and receives the determination result of the image determination server 20 as to whether or not the captured image is the meal image and the server information organization unit 95 as an information organization unit that organizes the captured image stored in the server storage unit 100. The server information organization unit 95 deletes the captured image from the server storage unit 100 when the determination communication unit 90 receives the determination result indicating that the captured image is not the meal image, and the server storage unit 100 holds the captured image when the determination communication unit 90 receives the determination result indicating that the captured image is the meal image.

According to such a configuration, because the determination communication unit 90 is located in the information processing server 16 rather than the wearable device 12, in comparison with the case where the determination communication unit 90 is provided in the wearable device 12, the consumption of the battery 54 of the wearable device 12 can be prevented.

The wearable device 12 includes the storage unit 82 that stores the captured image taken by the camera 48 and the device information organization unit 84 that organizes the captured image stored in the storage unit 82. After transmitting the captured image stored in the storage unit 82 to the information processing server 16, the device information organization unit 84 deletes the captured image from the storage unit 82.

According to such a configuration, the storage capacity of the storage unit 82 required for storing the captured image can be reduced.

The wearable device 12 includes the light emitting unit 50 that outputs the guide light L in the image-capturing range of the camera 48.

According to such a configuration, the user 200 can easily fit the subject 202 within the image-capturing range of the camera 48 by irradiating the subject 202 (meal) with the guide light L at the time of image-capturing.

The wearable device 12 includes the two operation switches 58 that can be operated by the user 200 and the light emitting control unit 78 that controls an operation of the light emitting unit 50. The light emitting control unit 78 outputs the guide light L from the light emitting unit 50 when the two operation switches 58 are operated at the same time and does not output the guide light L from the light emitting unit 50 when only one of the two operation switches 58 is operated.

According to such a configuration, it is possible to prevent the guide light L from being output from the light emitting unit 50 when any one of the two operation switches 58 is erroneously operated.

The wearable device 12 includes the subject determination unit 79 that determines whether or not the subject 202 entering the image-capturing range of the camera 48 corresponds to predetermined meal information and the camera control unit 80 that controls an operation of the camera 48. The camera control unit 80 prohibits the camera 48 from image-capturing the subject 202 when the subject determination unit 79 determines that the subject 202 does not correspond to the predetermined meal information and permits the camera 48 to image-capture the subject 202 when the subject determination unit 79 determines that the subject 202 corresponds to the predetermined meal information.

According to such a configuration, it is possible to effectively prevent the camera 48 of the wearable device 12 from capturing an image (for example, capturing a voyeur image) that is not necessary for health management.

The wearable device 12 includes the notification control unit 86 that performs notification control for prompting the user 200 to input the meal date time. The information processing server 16 includes: the date time acquisition determination unit 93 that determines whether or not the meal date time of the user 200 has been acquired:; the time determination unit 98 that determines whether or not the elapsed time T1 from the previous meal date time exceeds the predetermined meal interval T2 when the date time acquisition determination unit 93 determines that the meal date time has not been acquired; and the notification request control unit 94 that requests the wearable device 12 to perform the notification control when the time determination unit 98 determines that the elapsed time T1 from the previous meal date time exceeds the predetermined meal interval T2.

With such a configuration, it is possible to effectively acquire the meal date time of the user 200.

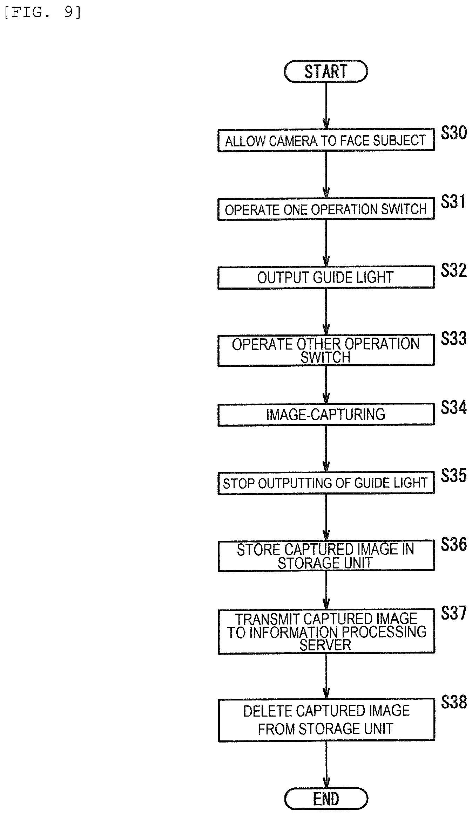

Next, the operation flow of the wearable device 12 according to Modified Example 1 will be described. As illustrated in FIG. 9, in this case, the user 200 operates one of the operation switches 58 (step S31) in the state where the camera 48 of the wearable device 12 faces the subject 202 (step S30). Then, the light emitting control unit 78 outputs the guide light L from the light emitting unit 50 (step S32).

Subsequently, when the user 200 operates the other operation switch 58 (step S33), the camera control unit 80 capture an image of the subject 202 (step S34). Then, when the image-capturing of the subject 202 by the camera 48 is completed, the light emitting control unit 78 stops the outputting of the guide light L from the light emitting unit 50 (step S35). That is, when the image-capturing of the subject 202 is completed, the outputting of the guide light L is automatically stopped without operating the operation switch 58. Accordingly, the user 200 can easily know that the image-capturing is completed.

After that, the processes of steps S36 to S38 are performed. It is noted that, because the processes of steps S36 to S38 are the same as the processes of steps S5 to S7 of FIG. 5 described above, the description thereof will be omitted.

In the wearable device 12 used for such an operation flow, the subject determination unit 79 described above is deleted. The same applies to the wearable device 12 used in the operation flow according to Modified Example 2 described later.

In the operation flow of the wearable device 12 according to Modified Example 1, the light emitting control unit 78 outputs the guide light L from the light emitting unit 50 when one of the two operation switches 58 is operated, and the camera control unit 80 allows the camera 48 to perform image-capturing when the other of the two operation switches 58 is operated.

According to such a configuration, the user 200 can perform the image-capturing by the camera 48 at the user's own timing.

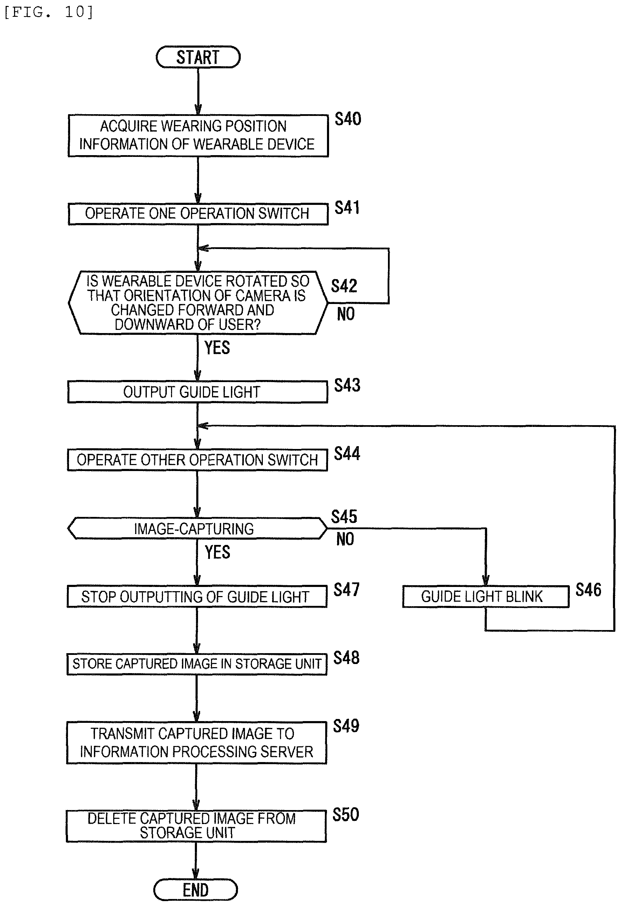

Next, the operation flow of the wearable device 12 according to Modified Example 2 will be described. As illustrated in FIG. 10, in this case, the wearable device 12 acquires wearing position information of the wearable device 12 of the user 200 (step S40). Specifically, the user 200 inputs information on which of the right and left arms the wearable device 12 is worn to the information processing device 14. Then, because the information is transmitted from the information processing device 14 to the wearable device 12, the wearable device 12 acquires the wearing position information of the wearable device 12 of the user 200.

Subsequently, the user 200 operates one of the operation switches 58 (step S41). At this time, the control unit 66 determines whether or not the wearable device 12 is rotated so that the orientation of the camera 48 is changed forward and downward of the user 200 (step S42). Specifically, the control unit 66 determines the rotation direction of the wearable device 12 based on the wearing position information of the wearable device 12 acquired in step S41 and the output signal of the acceleration sensor 70.

When the control unit 66 determines that the wearable device 12 is not rotated so that the orientation of the camera 48 is changed forward and downward of the user 200 (step S42: NO), the process stays in step S42 until the wearable device 12 is rotated so that the orientation of the camera 48 is changed forward and downward of the user 200.

On the other hand, when the control unit 66 determines that the wearable device 12 is rotated so that the orientation of the camera 48 is changed forward and downward of the user 200 (step S42: YES), the light emitting control unit 78 outputs the guide light L from the light emitting unit 50 (refer to step S43, FIGS. 11A and 11B).

Subsequently, when the user 200 operates the other operation switch 58 (step S44), the camera control unit 80 captures an image of the subject 202 (step S45). Specifically, the camera control unit 80 permits the camera 48 to perform image-capturing when the orientation of the camera 48 is obliquely downward and prohibits the camera 48 to perform image-capturing when the orientation of the camera 48 is not obliquely downward (for example, when the orientation of the camera 48 is obliquely upward).

When the image-capturing of the subject 202 cannot be performed (step S45: NO), the light emitting control unit 78 controls an operation of the light emitting unit 50 so that the guide light L blinks (step S46). Accordingly, it is possible to notify the user 200 that the image-capturing of the subject 202 has not been completed. However, in step S46, the light emitting control unit 78 is not limited to the example of changing the output pattern of the guide light L, and may control the operation of the light emitting unit 50 so that the color of the guide light L is changed. In this case, the process returns to step S44.

Then, when the image-capturing of the subject 202 by the camera 48 is completed (step S45: YES), the light emitting control unit 78 stops the outputting of the guide light L from the light emitting unit 50 (step S47). That is, when the image-capturing of the subject 202 is completed, the outputting of the guide light L is automatically stopped without operating the operation switches 58. Accordingly, the user 200 can easily know that the image-capturing is completed.

After that, the processes of steps S48 to S50 are performed. It is noted that, because the processes of steps S48 to S50 are the same as the processes of steps S5 to S7 of FIG. 5 described above, the description thereof will be omitted.

In the operation flow of the wearable device 12 according to Modified Example 2, the camera control unit 80 permits the camera 48 to image-capture the subject 202 when the orientation of the camera 48 is obliquely downward and prohibits the camera 48 from image-capturing the subject 202 when the orientation of the camera 48 is not obliquely downward.

According to such a configuration, because the image-capturing range can be narrowed according to the positional relationship between the wearable device 12 and the user 200, it is possible to prevent the camera 48 from being abused (used for capturing a voyeur image).

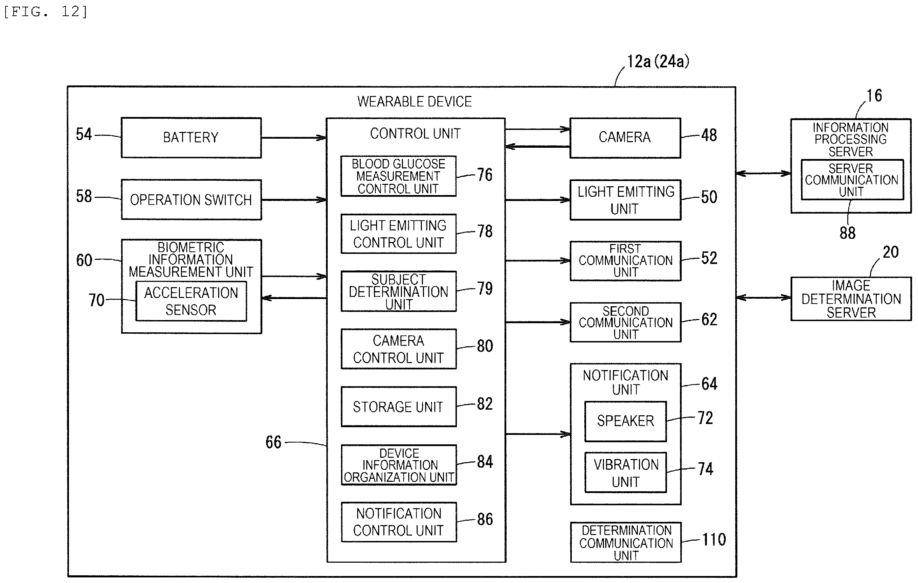

The image management system 10 may include a wearable device 12a illustrated in FIG. 12 instead of the wearable device 12 described above. In the description of the wearable device 12a, the same reference numerals are denoted to the same configurations as the above-mentioned wearable device 12, and the description thereof will be omitted.

As illustrated in FIG. 12, a device main body 24a of the wearable device 12a includes a determination communication unit 110. In this case, the above-mentioned determination communication unit 90 of the information processing server 16 is deleted. The information processing server 16 cooperates with the coaching device 22 in the same manner as in FIG. 1.

The determination communication unit 110 constructs a wireless communication line between the determination communication unit 110 and the image determination server 20 to perform information communication. As the standard of the wireless communication line between the determination communication unit 110 and the image determination server 20, the same standard as the standard of the wireless communication line between the first communication unit 52 and the server communication unit 88 can be used.

The determination communication unit 110 transmits the captured image stored in the storage unit 82 to the image determination server 20 and receives the determination result of the image determination server 20 as to whether or not the captured image is the meal image. The device information organization unit 84 deletes the captured image from the storage unit 82 when the determination communication unit 110 receives the determination result indicating that the captured image is not the meal image and transmits the captured image to the information processing server 16 and, after that, deletes the captured image from the storage unit 82 when the determination communication unit 110 receives the determination result indicating that the captured image is the meal image.

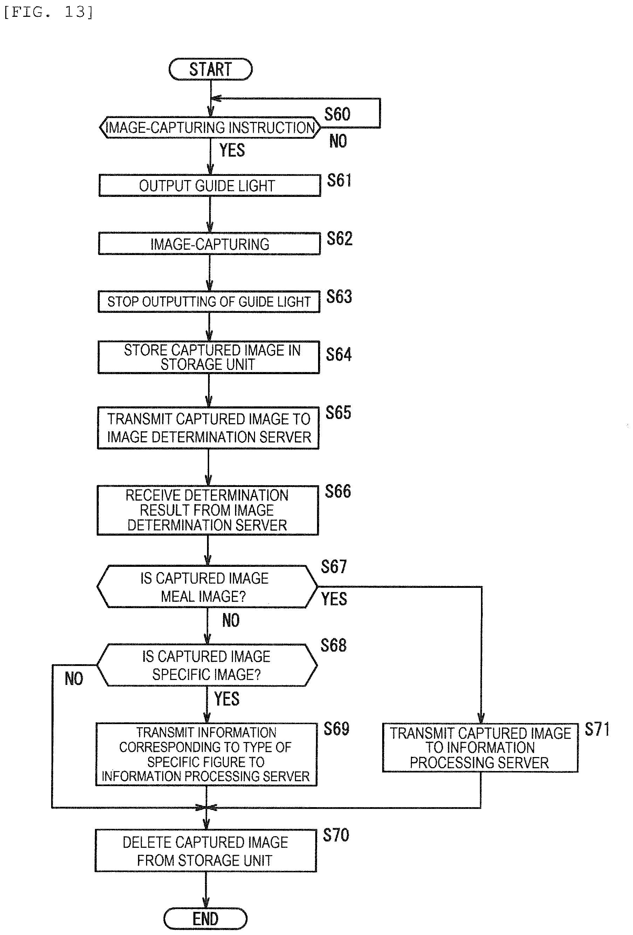

Next, the operation flow of the wearable device 12a will be described. As illustrated in FIG. 13, because the processes from step S60 to step S64 are the same as the processes from step S1 to step S5 in FIG. 5 described above, the description thereof will be omitted.

After step S64, the determination communication unit 110 transmits the captured image stored in the storage unit 82 to the image determination server 20 (step S65). Then, the image determination server 20 determines whether or not the captured image is the meal image or the specific image and transmits the determination result to the determination communication unit 110. Then, the determination communication unit 110 receives the determination result as to whether or not the captured image is the meal image (step S66).

Next, when the determination communication unit 110 receives the determination result indicating that the captured image is not the meal image (step S67: NO), it is determined whether or not the captured image includes the specific image (step S68). When the captured image includes the specific image (step S68: YES), the device information organization unit 84 transmits information corresponding to a type of the specific figure included in the captured image to the information processing server 16 (step S69). After that, the device information organization unit 84 deletes the specific image from the storage unit 82 (step S70). When the captured image does not include a specific image (step S68: NO), the captured image is deleted from the storage unit 82 (step S70). Accordingly, the storage capacity of the storage unit 82 required to store the captured image can be reduced, and the information corresponding to the specific image to be acquired can be efficiently collected. In addition, even when an image (for example, a voyeur image or the like) other than the meal image or the necessary specific image is captured by the wearable device 12a, the captured image can be deleted from the storage unit 82. After that, the operation flow of this time is terminated.

When the determination communication unit 110 receives the determination result indicating that the captured image is the meal image (step S67: YES), the device information organization unit 84 transmits the captured image stored in the storage unit 82 to the information processing server 16 (step S71). After that, the device information organization unit 84 deletes the captured image from the storage unit 82 (step S70). Then, the operation flow of this time is terminated. It is noted that, in the information processing server 16, processes are performed according to the flowcharts of FIGS. 6 and 7 described above (however, in this case, steps S16 to S19 are not performed).

Such an image management method includes an image-capturing step (step S62) of image-capturing the subject 202 by the camera 48 of the wearable device 12a worn by the user 200, the storing step (step S64) of storing the captured image taken in the image-capturing step in the storage unit 82 of the wearable device 12a, an image determination step (step S65 and step S66) of transmitting the captured image stored in the storage unit 82 to the image determination server 20 and receiving the determination result of the image determination server 20 as to whether or not the captured image is the meal image, and an information organizing step (step S70 and step S71) of organizing the captured image stored in the storage unit 82, and in the information organizing step, the captured image is deleted from the storage unit 82 when the wearable device 12a receives the determination result indicating that the captured image is not the meal image, and the captured image is deleted after the captured image is transmitted to the information processing server 16 when the wearable device 12a receives the determination result indicating that the captured image is the meal image.

Such a wearable device 12a includes the camera 48 for image-capturing the subject 202, the storage unit 82 that stores the captured image taken by the camera 48, the determination communication unit 110 that transmits the captured image stored in the storage unit 82 to the image determination server 20 and receives the determination result of the image determination server 20 according to whether or not the captured image is the meal image, and the device information organization unit 84 that organizes captured image stored in the storage unit 82. The device information organization unit 84 deletes the captured image from the storage unit 82 when the determination communication unit 110 receives the determination result indicating that the captured image is not the meal image and deletes the captured image after transmitting the captured image to the information processing server 16 when the determination communication unit 110 receives the determination result indicating that the captured image is the meal image. The information transmitted to the information processing server 16 is provided to the coaching device 22.

According to such a configuration, when the captured image is not the meal image, the captured image is deleted from the storage unit 82, and thus, health management using the meal image can be effectively performed. The wearable device 12a has the same effect as the above-mentioned wearable device 12.

The invention is not limited to the above-described embodiment, and various modifications can be made without departing from the spirit of the invention.

The information processing server 16 may request at least one of the wearable device 12 and 12a and the information processing device 14 to control the blood glucose measurement notification to prompt to measure the blood glucose after a lapse of a certain time from the user 200 image-capturing of the meal.

The above embodiments can be summarized as follows.

The above-described embodiment discloses an image management system (10) for medical use including: a wearable device (12, 12a) worn by a user (200); and an information processing server (16) configured to communicate with the wearable device, wherein the wearable device includes a camera (48) for image-capturing a subject (202), wherein the information processing server includes a server storage unit (100) that stores a captured image received from the wearable device, wherein any one of the wearable device and the information processing server includes an information organization unit (84, 95) that organizes the captured image based on a determination result of an image determination unit as to whether or not the captured image taken by the camera is a meal image, wherein the information organization unit deletes the captured image when the image determination unit determines that the captured image is not the meal image, and wherein the server storage unit holds the captured image when the image determination unit determines that the captured image is the meal image.

In the above-described image management system, the image determination unit may be an image determination server (20) provided separately from the wearable device and the information processing server, and the information processing server may include a determination communication unit (90) that transmits the captured image stored in the server storage unit to an image determination server and receives a determination result of the image determination server as to whether or not the captured image is a meal image, and a server information organization unit (95) as the information organization unit that organizes the captured image stored in the server storage unit, wherein the server information organization unit deletes the captured image from the server storage unit when the determination communication unit receives the determination result indicating that the captured image is not the meal image, and wherein the server storage unit holds the captured image when the determination communication unit receives the determination result indicating that the captured image is the meal image.

In the above-described image management system, the wearable device may include a storage unit (82) that stores the captured image taken by the camera and a device information organization unit (84) that organizes the captured image stored in the storage unit, and the device information organization unit may delete the captured image from the storage unit after transmitting the captured image stored in the storage unit to the information processing server.

In the above-described image management system, the wearable device may include a light emitting unit (50) that outputs the guide light (L) in the image-capturing range of the camera.

In the above-described image management system, the wearable device may include two operation units (58) that can be operated by the user and a light emitting control unit (78) that controls an operation of the light emitting unit, and the light emitting control unit may output the guide light from the light emitting unit when the two operation units are operated at the same time and may not output the guide light from the light emitting unit when only one of the two operation units is operated.

In the above-described image management system, the wearable device may include a subject determination unit (79) that determines whether or not the subject (202) entering the image-capturing range of the camera corresponds to the predetermined meal information and a camera control unit (80) that controls an operation of the camera, and the camera control unit may prohibit the camera from image-capturing the subject when the subject determination unit determines that the subject does not correspond to the predetermined meal information and may permit the camera to image-capture the subject when the subject determination unit determines that the subject corresponds to the predetermined meal information.

In the above-described image management system, the wearable device may include two operation units that can be operated by the user, a light emitting control unit that controls an operation of the light emitting unit, and a camera control unit that controls an operation of the camera, and the light emitting control unit may output the guide light from the light emitting unit when one of the two operation units is operated, and the camera control unit may allow the camera to perform image-capturing when the other of the two operation units is operated.

In the above-described image management system, the wearable device may include a camera control unit that controls an operation of the camera, and the camera control unit may permit the camera to image-capture the subject when the orientation of the camera is obliquely downward and prohibit the camera from performing image-capturing when the orientation of the camera is not obliquely downward.

In the above-described image management system, the wearable device may include a notification control unit (86) that performs notification control for prompting the user to input the meal date time, and the information processing server may include: a date time acquisition determination unit (93) that determines whether or not the meal date time of the user have been acquired; a time determination unit (98) that determines whether or not the elapsed time (T1) from the previous meal date time exceeds the predetermined meal interval (T2) when the date time acquisition determination unit determines that the meal date time have not been acquired; and a notification request control unit (94) that requests the wearable device to perform the notification control when the time determination unit determines that the elapsed time from the previous meal date time exceeds the predetermined meal interval.

The embodiment discloses a wearable device (12a) for medical use worn by a user including: a camera for performing image-capturing a subject; a storage unit that stores a captured image taken by the camera; a determination communication unit (110) that transmits the captured image stored in the storage unit to an image determination server and receives a determination result of the image determination server as to whether or not the captured image is a meal image; and a device information organization unit that organizes the captured image stored in the storage unit, and the device information organization unit deletes captured image from the storage unit when the determination communication unit receives the determination result indicating that the captured image is not the meal image and deletes captured image from the storage unit after transmitting captured image to the information processing serve when the determination communication unit receives the determination result indicating that the captured image is the meal image.

In the above-described wearable device, a light emitting unit for outputting the guide light to the subject may be provided.

The wearable device may include two operation units that can be operated by the user and a light emitting control unit that controls an operation of the light emitting unit, and the light emitting control unit may output the guide light from the light emitting unit when the two operation units are operated at the same time and may not output the guide light from the light emitting unit when only one of the two operation units is operated.

The above-described wearable device may include: a subject determination unit that determines whether or not the subject entering the image-capturing range of the camera is the same as or similar to the predetermined meal image and a camera control unit that controls an operation of the camera, and the camera control unit may prohibit the camera from image-capturing when the subject determination unit determines that the subject does not correspond to the predetermined meal information and may permit the camera to image-capture when the subject determination unit determines that the subject corresponds to the predetermined meal information.

The wearable device may include two operation units that can be operated by the user, a light emitting control unit that controls an operation of the light emitting unit, and a camera control unit that controls an operation of the camera, and the light emitting control unit may output the guide light from the light emitting unit when one of the two operation units is operated, and the camera control unit may allow the camera to perform image-capturing when the other of the two operation units is operated.

In the above-described wearable device, the wearable device may include a mounting unit (26, 28) for mounting the wearable device on the wrist of the user and a camera control unit for controlling an operation of the camera, and the camera control unit may allow the camera to perform image-capturing by rotating the wearable device so that the image-capturing range of the camera faces downward.

The above embodiment discloses an image management method for medical use including: an image-capturing step of image-capturing a subject by a camera of a wearable device worn by a user; an image transmitting step of transmitting a captured image taken in the image-capturing step to an information processing server; a storing step of storing the captured image received from the wearable device in a server storage unit of the information processing server; and a server information organizing step of organizing the captured image based on a determination result of an image determination unit as to whether or not the captured image taken by the camera is a meal image, and in the server information organizing step, the captured image is deleted when the image determination unit determines that the captured image is not the meal image, and the captured image is held in the server storage unit when the image determination unit determines that the captured image is the meal image.

The above embodiment discloses is an image management method for medical use including: an image-capturing step of image-capturing a subject by a camera of a wearable device worn by a user, a storing step of storing a captured image taken in the image-capturing step in a storage unit of the wearable device, an image determination step of transmitting the captured image stored in the storage unit to an image determination server and receiving a determination result of the image determination server as to whether or not the captured image is a meal image, and an information organizing step of organizing the captured image stored in the storage unit, and in the information organizing step, the captured image is deleted from the storage unit when the wearable device receives the determination result indicating that the captured image is not the meal image, and the captured image is deleted after the captured image is transmitted to an information processing server when the wearable device receives the determination result indicating that the captured image is the meal image.

The above embodiment discloses an image management program for medical use causing a computer to execute: an image-capturing step of image-capturing a subject by a camera of a wearable device worn by a user; an image transmitting step of transmitting a captured image taken in the image-capturing step to an information processing server; a storing step of storing the captured image received from the wearable device in a server storage unit of the information processing server; and a server information organizing step of organizing the captured image based on a determination result of an image determination unit as to whether or not the captured image taken by the camera is a meal image, in the server information organizing step, the captured image is deleted when the image determination unit determines that the captured image is not the meal image, and the captured image is held in the server storage unit when the image determination unit determines that the captured image is the meal image.

The above embodiment discloses an image management program for medical use causing a computer to execute: an image-capturing step of image-capturing a subject by a camera of a wearable device worn by a user; a storing step of storing the captured image taken in the image-capturing step in a storage unit of the wearable device; and an image determination step of transmitting the captured image stored in the storage unit to an image determination server and receiving the determination result of the image determination server as to whether or not the captured image is a meal image, and an information organizing step of organizing the captured image stored in the storage unit, and in the information organizing step, the captured image is deleted from the storage unit when the wearable device receives the determination result indicating that the captured image is not the meal image, and the captured image is deleted after the captured image is transmitted to an information processing server when the wearable device receives the determination result indicating that the captured image is the meal image.

## Claims

1. An image management system for medical use, comprising:
a wearable device worn by a user; and
an information processing server communicatable with the wearable device,
wherein the wearable device comprises a camera for image-capturing a subject,
wherein the information processing server comprises a server storage unit that stores a captured image received from the wearable device,
wherein any one of the wearable device and the information processing server includes an information organization unit that organizes the captured image based on a determination result of an image determination unit as to whether or not the captured image taken by the camera is a meal image,
wherein the information organization unit deletes the captured image when the image determination unit determines that the captured image is not the meal image, and
wherein the server storage unit holds the captured image when the image determination unit determines that the captured image is the meal image.

2. The image management system according to claim 1,
wherein the image determination unit is an image determination server provided separately from the wearable device and the information processing server,
wherein the information processing server includes:
a determination communication unit that transmits the captured image stored in the server storage unit to the image determination server and receives a determination result of the image determination server as to whether or not the captured image is a meal image; and
a server information organization unit as the information organization unit that organizes the captured image stored in the server storage unit,
wherein the server information organization unit deletes the captured image from the server storage unit when the determination communication unit receives the determination result indicating that the captured image is not the meal image, and
wherein the server storage unit holds the captured image when the determination communication unit receives the determination result indicating that the captured image is the meal image.

3. The image management system according to claim 2,
wherein the wearable device comprises:
a storage unit that stores the captured image taken by the camera; and
a device information organization unit that organizes the captured image stored in the storage unit,
wherein the device information organization unit deletes the captured image from the storage unit after transmitting the captured image stored in the storage unit to the information processing server.

4. The image management system according to claim 2 or 3,
wherein the wearable device comprises a light emitting unit that outputs guide light in an image-capturing range of the camera.

5. The image management system according to claim 4,
wherein the wearable device comprises:
two operation units that can be operated by the user; and
a light emitting control unit that controls an operation of the light emitting unit, and
wherein the light emitting control unit outputs the guide light from the light emitting unit when the two operation units are operated at the same time, and does not output the guide light from the light emitting unit when only one of the two operation units is operated.

6. The image management system according to any one of claims 1 to 5,
wherein the wearable device comprises:
a subject determination unit that determines whether or not the subject entering the image-capturing range of the camera corresponds to predetermined meal information; and
a camera control unit that controls an operation of the camera, and
wherein the camera control unit prohibits the camera from image-capturing the subject when the subject determination unit determines that the subject does not correspond to the predetermined meal information and permits the camera to image-capture the subject when the subject determination unit determines that the subject corresponds to the predetermined meal information.

7. The image management system according to any one of claims 1 to 6,
wherein the wearable device comprises a notification control unit that performs notification control for prompting the user to input a meal date time, and
wherein the information processing server comprises:
a date time acquisition determination unit that determines whether or not the meal date time of the user has been acquired;
a time determination unit that determines whether or not an elapsed time from a previous meal date time exceeds a predetermined meal interval when the date time acquisition determination unit determines that the meal date time has not been acquired; and
a notification request control unit that requests the wearable device to perform the notification control when the time determination unit determines that the elapsed time from the previous meal date time exceeds the predetermined meal interval.

8. A wearable device for medical use worn by a user, comprising:
a camera for image-capturing a subject;
a storage unit that stores a captured image taken by the camera;
a determination communication unit that transmits the captured image stored in the storage unit to an image determination server and receives a determination result of the image determination server as to whether or not the captured image is a meal image; and
a device information organization unit that organizes the captured image stored in the storage unit,
wherein the device information organization unit deletes the captured image from the storage unit when the determination communication unit receives the determination result indicating that the captured image is not the meal image and deletes the captured image from the storage unit after transmitting the captured image to an information processing server when the determination communication unit receives the determination result indicating that the captured image is the meal image.

9. The wearable device according to claim 8, comprising a light emitting unit for outputting guide light to the subject.

10. The wearable device according to claim 9, comprising:
two operation units that can be operated by the user;
a light emitting control unit that controls an operation of the light emitting unit,
wherein the light emitting control unit outputs the guide light from the light emitting unit when the two operation units are operated at the same time and does not output the guide light from the light emitting unit when only one of the two operation units is operated.

11. The wearable device according to any one of claims 9 to 10, comprising:
a subject determination unit that determines whether or not the subject entering an image-capturing range of the camera is the same as or similar to a predetermined meal image; and
a camera control unit that controls an operation of the camera,
wherein the camera control unit prohibits the camera from capturing the image when the subject determination unit determines that the subject does not correspond to predetermined meal information and permits the camera to capture the image when the subject determination unit determines that the subject corresponds to the predetermined meal information.

12. The wearable device according to claim 9, comprising:
two operation units that can be operated by the user;
a light emitting control unit that controls an operation of the light emitting unit; and
a camera control unit that controls an operation of the camera,
wherein the light emitting control unit outputs the guide light from the light emitting unit when one of the two operation units is operated, and
wherein the camera control unit allows the camera to capture the image when the other of the two operation units is operated.

13. The wearable device according to any one of claims 8 to 12, comprising:
a mounting unit for mounting the wearable device on a wrist of the user; and
a camera control unit that controls an operation of the camera,
wherein the camera control unit allows the camera to capture the image when the wearable device is rotated so that the image-capturing range of the camera faces downward.

14. An image management method for medical use, comprising:
an image-capturing step of capturing an image of a subject using a camera of a wearable device worn by a user;
an image transmitting step of transmitting a captured image taken in the image-capturing step to an information processing server;
a storing step of storing the captured image received from the wearable device in a server storage unit of the information processing server; and
a server information organizing step of organizing the captured image based on the determination result of an image determination unit as to whether or not the captured image taken by the camera is a meal image,
wherein, in the server information organizing step, when the image determination unit determines that the captured image is not the meal image, the captured image is deleted, and when the image determination unit determines that the captured image is the meal image, the captured image is held in the server storage unit.

15. An image management method for medical use, comprising:
an image-capturing step of capturing an image of a subject by a camera of a wearable device worn by a user;
a storing step of storing a captured image taken in the image-capturing step in a storage unit of the wearable device;
an image determination step of transmitting the captured image stored in the storage unit to an image determination server and receiving a determination result of the image determination server as to whether or not the captured image is a meal image; and
an information organizing step of organizing the captured image stored in the storage unit,
wherein, in the information organizing step, the captured image is deleted from the storage unit when the wearable device receives the determination result indicating that the captured image is not the meal image, and the captured image is deleted after the captured image is transmitted to an information processing server when the wearable device receives the determination result indicating that the captured image is the meal image.

16. An image management program for medical use, causing a computer to execute:
an image-capturing step of capturing an image of a subject by a camera of a wearable device worn by a user;
an image transmitting step of transmitting a captured image taken in the image-capturing step to an information processing server;
a storing step of storing the captured image received from the wearable device in a server storage unit of the information processing server; and
a server information organizing step of organizing the captured image based on a determination result of an image determination unit as to whether or not the captured image taken by the camera is a meal image,
wherein, in the server information organizing step, the captured image is deleted when the image determination unit determines that the captured image is not the meal image, and the captured image is held in the server storage unit when the image determination unit determines that the captured image is the meal image.

17. An image management program for medical use, causing a computer to execute:
an image-capturing step of capturing an image of a subject using a camera of a wearable device worn by a user;
a storing step of storing a captured image taken in the image-capturing step in a storage unit of the wearable device;
an image determination step of transmitting the captured image stored in the storage unit to an image determination server and receiving a determination result of the image determination server as to whether or not the captured image is a meal image; and
an information organizing step of organizing the captured image stored in the storage unit,
wherein, in the information organizing step, the captured image is deleted from the storage unit when the wearable device receives the determination result indicating that the captured image is not the meal image, and the captured image is deleted after the captured image is transmitted to an image processing server when the wearable device receives the determination result indicating that the captured image is the meal image.
